# EUROPEAN PATENT APPLICATION

(11) **EP 2 319 535 A2**
(43) Date of publication of application: **11.05.2011**
(21) Application number: 10178622.6
(22) Date of filing: 20.10.1995
(51) Int. Cl.: A61K 39/395, A61P 13/12, A61P 3/00

(54) **Pharmaceutical composition for treatment of diseases caused by IL-6 production**

(30) Priority: 21.10.1994 JP 25701094
(62) Divisional of application: 07021532.2
(71) Applicant: Chugai Seiyaku Kabushiki Kaisha, Tokyo 115 (JP); Kishimoto, Tadami, Tondabayashi-shi, Osaka 584 (JP)
(72) Inventor: Kishimoto, Tadamitsu, Osaka 584 (JP); Katsume, Asao, Shizuoka 412 (JP); Saito, Hiroyuki, Shizuoka 412 (JP)
(74) Representative: Wakerley, Helen Rachael

(57) **Abstract**

Pharmaceutical compositions for prevention or treatment of diseases caused by interleukin-6 production, comprising an antibody to interleukin-6 receptor (IL-6R antibody). As the IL-6R antibody, an antibody of animals other than the human such as mice, rats, etc., a chimeric antibody between these and a human antibody, a reshaped human antibody, etc. may be used.

The pharmaceutical compositions are useful for prevention or treatment of diseases caused by interleukin-6 production such as plasmacytosis, anti-IgG1-emia, anemia, nephritis, etc,

## Description

### Technical Field

The present invention relates to pharmaceutical compositions for prevention or treatment of diseases caused by interleukin-6 (IL-6) production, comprising an antibody (anti-IL-6R antibody) to interleukin-6 receptor (IL-6R).

### Background Art

IL-6 is a multi-functional cytokine that is believed to work at various stages of immunological, hematological, and acute-phase reactions [Taga, T. et al., Critical Reviews in Immunol. 11:265-280, 1992], and to play important roles in multiple myeloma as a growth factor as well as in diseases which are accompanied by plasmacytosis such as rheumatism [Hirano, T. et al., Eur. J. Immunol. 18:1797-1801, 1988; Houssiau, F.A. et al., Arth. Rheum. 31:784-788, 1988], in Castleman's disease [Yoshizaki, K. et al., Blood 74:1360-1367, 1989; Brant, S.J. et al., J. Clin. Invest. 86:592-599, 1990], mesangium cell proliferative nephritis [Ohta, K. et al., Clin. Nephrol. (Germany ) 38:185-189, 1992; Fukatsu, A. et al., Lab. Invest. 65:61-66, 1991; Horii, Y. et al., J. Immunol. 143:3949-3955, 1989], cachexia accompanied by tumor-growth [Strassmann, G. et al., J. Clin. Invest. 89:1681-1684, 1992], etc.

In H-2 L^{d} hIL-6 transgenic mouse (IL-6 Tgm) that has expressed human IL-6 (hIL-6) in excessive levels by genetic engineering, IgG1 plasmacytosis, mesangium cell proliferative nephritis, anemia, thrombocytopenia, appearance of autoantibodies, etc. have been observed [Miyai, T. et al., a presentation at the 21st Meeting of Japan Immunology Society "Hematological change in H-2 Ld hIL-6 transgenic mice with age," 1991], suggesting the involvement of IL-6 in a variety of diseases. However, it is not known that antibody to interleukin-6 receptor is effective for diseases caused by interleukin production.

### Disclosure in Invention

Thus, in accordance with the present invention, there is provided a pharmaceutical composition for prevention or treatment of diseases caused by interleukin-6 production.

In order to resolve the above problems, the present invention provides pharmaceutical compositions for prevention or treatment of diseases caused by interleukin-6 production, said pharmaceutical compositions comprising an antibody to interleukin-6 receptor.

### Brief Explanation of Drawings

Fig. 1 is a graph showing change in increases in the body weight of animals in each group.
Fig. 2 is a graph showing change in positive ratio of urinary protein in each group. The positive ratio of urinary protein was zero' in the groups other than Group 1 and 3.
Fig. 3 is a graph showing change in hemoglobin level in each group.
Fig. 4 is a graph showing change in red blood cell count in each group.
Fig. 5 is a graph showing change in platelet count in each group.

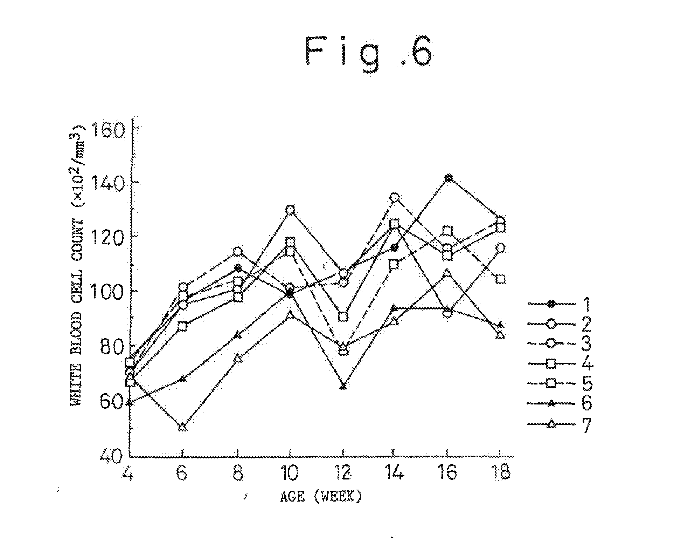

Fig. 6 is a graph showing change in white blood cell count in each group.
Fig. 7 is a graph showing change in IgGl concentration in serum in each group.
Fig. 8 is a graph showing change in human IL-6 concentration in Group 1 through 5.
Fig. 9 represents a result of cell sorting by a fluorescent antibody technique using the control antibody IgG and Gr-1 antibody in Group 1 and 2.
Fig. 10 represents a result of cell sorting by a fluorescent antibody technique using the control antibody IgG and Gr-1 antibody in Group 6 and 7.
Fig. 11 is a graph showing the weight of the spleen of the animals in each group at the end of the experiment.
Fig. 12 is a graph showing change in the body weight of the animals in each group.
Fig. 13 is a graph showing the concentration of triglyceride in the blood of the mice on day 11 of the experiment.
Fig. 14 is a graph showing the concentration of glucose in the blood of the mice on day 15 of the experiment.
Fig. 15 is a graph showing the concentration of ionized calcium in the blood of the mice on day 11 of the experiment.
Fig. 16 is a graph showing the survival rate of the tumor bearing control mice.
Fig. 17 is a graph showing the body weight of the mice on day 10 and 12 after the start of the experiment.
Fig. 18 is a graph showing the concentration of ionized calcium in the blood of the mice on day 10 and 12 after the start of the experiment.

### Specific Explanation

Diseases caused by interleukin-6 production include, for example, plasmacytosis such as rheumatism and Castleman's disease; hyperimmunoglobulinemia; anemia; nephritis such as mesangium proliferative nephritis; cachexia etc.

The antibody to interleukin-6 receptor to be used in the present invention may be of any origin or type (monoclonal, polyclonal) as long as it can block signal transduction by IL-6 and inhibit the biological activity of IL-6. Preferably, however, it is a monoclonal antibody derived from a mammal. The antibody blocks signal transduction by IL-6 and inhibits the biological activity of IL-6 by inhibiting the binding of IL-6 to IL-6R.

The animal species of the cell for producing the monoclonal antibody can be any animal species belonging to the mammals and may be human antibody or antibody derived from an animal other than the human. The monoclonal antibodies derived from an animal other than the human are preferably monoclonal antibodies derived from a rabbit or a rodent because of its ease of production. Preferably, the rodent includes, but not limited to, mice, rats, hamsters, etc.

Such an antibody to interleukin-6 receptor includes, for example, MR16-1 antibody (Tamura, T. et al., Proc. Natl. Acad. Sci. U.S.A. 90:11924-11928, 1993), PM-1 antibody (Hirata, Y. et al., J. Immunol. 143:2900-2906, 1989), etc.

The monoclonal antibodies may be produced essentially by the method known in the art as follows. Thus, they may be produced by using IL-6R as the immunizing antigen which is used for immunization by the conventional method, and then the immunocytes obtained are subjected to cell fusion with a known parent cell by the conventional cell fusion method to screen the antibody-producing cells by the conventional screening method.

More specifically the monoclonal antibodies are produced in the following method. For example, said immunizing antigen may be obtained by using the gene sequence of human IL-6R as set forth in European Patent Application EP 325474. After the gene sequence of human IL-6R is inserted into a known expression vector system to transform a suitable host cell, the desired IL-6R protein is purified from the host cells or the culture supernatant thereof to employ said purified IL-6R protein as the immunizing antigen.

Furthermore, said immunizing antigen derived from the mouse may be obtained using the gene sequence of the mouse IL-6R which was described in the Japanese Unexamined Patent Publication 3(1991)-155795 by the same method as used for the above-mentioned gene sequence of the human IL-6R.

As the IL-6R, in addition to those expressed on the cell membrane, those (sIL-6R) that are possibly detached from the cell membrane may be used as the antigen. sIL-6R is mainly composed of the extracellular domain of the IL-6R bound to the cell membrane, being different from the membrane-bound IL-6R in that the former lacks the transmembrane domain or both of the transmembrane domain and the intracellular domain.

Among the mammals immunized with the immunizing antigen are not necessarily limited, but it is preferable to take into consideration its compatibility with the parent cell used for cell fusion, and usually mice, rats, hamsters, rabbits, etc. are used.

Immunization of the animal with the immunizing antigen may be effected in accordance with a method known to those skilled in the art. A general method, for example, comprises administering intraperitoneally or subcutaneously said immunizing antigen to the mammal. Specifically, an immunizing antigen diluted or suspended in PBS (phosphate buffered saline), physiological saline, etc. to a suitable volume is mixed, as desired, with a suitable amount of an adjuvant such as complete Freund's adjuvant and is emulsified, and then preferably said emulsion is administered to a mammal several times every 4 to 21 days. Furthermore, a suitable carrier may be used at the time of immunization with the immunizing antigen.

After the animal was immunized as above and the antibody level in the serum was confirmed to have risen to the desired level, immunocytes are removed from the mammal and are subjected to cell fusion. As a preferred immunocyte, the spleen cell is particularly mentioned. The preferred myeloma cell used in the present invention as the partner parent cells that are fused with said immunocyte include various known cell lines, for example, P3 (p3x63Ag8.653) (J. Immunol. 123:1548,1978), p3-Ul (Current Topics i Micro-biology and Immunology 81:1-7, 1978), NS-1 (Eur. J. Immunol. 6:511-519, 1976), MPC-11 (Cell 8:405-415, 1976), SP2/0 (Nature 276:269-270, 1978), FO (J. Immunol. Meth. 35:1-21, 1980), S194 (J. Exp. Med. 148:313-323, 1978), R210 (Nature 277:131-133, 1979), etc.

Cell fusion of said immunocyte with the myeloma cell may be carried out essentially in accordance with a known method such as is described by Milstein et al. (Milstein et al., Methods Enzymol. 73:3-46, 1981), etc.

More specifically, said cell fusion may be carried out in the presence of, for example, a cell fusion accelerating agent in an ordinary nutrient medium. As the cell fusion accelerating agent, polyethylene glycol (PEG), Sendai virus (HVJ), etc. may be used, and an adjuvant such as dimethyl sulfoxide etc. may be directly added as desired in order to enhance the efficiency of cell fusion.

The ratio of the immunocytes to the myeloma cells used is preferably 1 to 10 times more immunocyte than the myeloma cells. As the liquid culture medium used for the above cell fusion, there are mentioned, for example, RPMI 1640 liquid medium and MEM liquid medium that are most suitable for growth of the myeloma cell line, and the common culture broths used for cell culture, and furthermore a serum supplement such as fetal calf serum (FCS) etc. may be used.

The desired fused cells (hybridoma) may be formed by mixing well a given amount of the above-mentioned immunocytes with the myeloma cells in the above-mentioned nutrient broth, and by adding a PEG solution previously warmed to 37°C, for example, a solution of PEG having an average molecular weight in the range of 1,000 to 6,000, at a concentration of 30 to 60% (w/v). Then after sequential addition of suitable culture media followed by centrifugation thereof to remove the supernatant, cell fusion agents etc. which are undesirable for growth of hybridoma can be removed.

Said hybridoma may be selected by culturing in a conventional selection medium such as, for example, HAT liquid culture medium (a liquid culture medium containing hypoxanthine, aminopterin, and thymidine). Culturing in said HAT medium is continued for a time period sufficient for the cells (non-fused cells) other than the desired hybridoma to die, usually for a few days to a few weeks. Subsequently a conventional limiting dilution method is carried out to screen and monoclone the hybridoma that produce the desired antibody.

The hybridoma that produces monoclonal antibodies thus prepared can be subcultured in a conventional liquid medium and stored in liquid nitrogen for a prolonged period of time.

In order to obtain a monoclonal antibody from said hybridoma, methods are employed such as the one in which said hybridoma is cultured in accordance with the conventional method to obtain a culture supernatant, or the one in which the hybridoma is implanted to and grown in a mammal compatible therewith followed by obtaining the antibody as the ascites fluid, and the like. The former method is suitable for obtaining a high-purity antibody, whereas the latter method is suitable for production of antibody in a large amount.

Furthermore, the monoclonal antibodies obtained by the above methods may be purified by the conventional procedures for purification such as salting-out, gel filtration, affinity chromatography, etc.

The ability of the thus prepared monoclonal antibodies to recognize the antigen with a high affinity and high precision can be confirmed by the conventional immunological methods such as the radioimmunoassay, the enzymeimmunoassay (EIA, ELISA), the fluorescent antibody method (immunofluorescence analysis), etc.

The monoclonal antibody used in the present invention is not limited to the monoclonal antibody produced by a hybridoma and can be an artificially altered one for the purpose of reducing heteroantigenicity to the human. For example, a chimera antibody comprising variable regions of a mouse monoclonal antibody and constant regions of a human antibody can be used. Such a chimera antibody may be produced using a known method for producing chimera antibodies, especially a genetic engineering method.

Furthermore, a reshaped human antibody can be used in the present invention. This is an antibody in which the complementarity determining regions of a human antibody has been replaced by the complementarity determining regions of a mammal antibody other than human antibody, e.g. a mouse antibody, and the general method of genetic engineering therefor are known in the art. Using such a known method, a reshaped human antibody can be obtained that is useful for the present invention.

As necessary, amino acids in the framework regions (FR) of the variable region of an antibody can be substituted so that the complementarity determining region of a reconstituted human antibody may form an appropriate antigen binding site (Sato et al., Cancer Res. 53:1-6, 1993). As such a reshaped human antibody, a humanized PM-1 (hPM-1) antibody may be preferably exemplified (see International Patent Application WO 9219759).

Genes encoding the fragments of an antibody, for example Fab or Fv, a single chain Fv (scFv) wherein the Fv's of an H chain and an L chain have been joined by a suitable linker, can be constructed and expressed in suitable host cells, and can be used for the above-mentioned purpose, as long as the fragments bind to the antigen and inhibit the-activity of IL-6 (see, for example, Bird et al., TIBTECH 9:132-137, 1991; Huston et al., Proc. Natl. Acad. Sci. U.S.A. 85:5879-5883, 1988). Furthermore, the above reshaped V region of the antibody can be used for Fv of the H chain and the L chain to make an scFv.

Pharmaceutical compositions for prevention or treatment of diseases caused by IL-6 production having the antibody to IL-6 receptor of the present invention as the active component may be used in the present invention, as long as they block signal transmission of IL-6 and are effective against diseases caused by IL-6 production.

The pharmaceutical compositions for prevention or treatment of diseases caused by IL-6 production may be preferably administered parenterally, for example via intravenous, intramuscular, intraperitoneal, or subcutaneous injection, etc., both systemically and locally. Furthermore, they can take a form of a pharmaceutical composition or a kit in combination with at least one pharmaceutical carrier or diluent.

Although dosage of the pharmaceutical compositions of the present invention may vary depending on the patient's disease conditions, age, or the method of administration, it is necessary to select a suitable amount as appropriate. For example, an amount in the range of 1 to 1,000 mg per patient may be given in up to four divided doses. Alternatively, they may be administered in an amount of 1 to 10 mg/kg/week. However, the pharmaceutical compositions of the present invention for prevention or treatment are not restricted to the above-mentioned doses.

The pharmaceutical compositions of the present invention may be formulated in the conventional method. For example, parenteral preparations may be prepared by dissolving a purified IL-6R antibody into a solvent, e.g. physiological saline, buffer solution etc., to which are added, anti-adsorption agent-e.g. Tween 80, gelatin, human serum albumin (HSA), etc., or they may be in a lyophilized form which may be reconstituted by dissolution prior to use. Excipients for lyophilization include, for example, a sugar alcohol such as mannitol, glucose, etc. or saccharides.

### EXAMPLES

The invention will now be explained in more detail with reference to the following reference examples and examples, but they must not be construed to limit the scope of the present invention.

### Reference Example 1. Construction of the B6L^{d}-IL-6 transgenic mouse

A 3.3 kbp of Sphl-XhoI fragment (L^{d}-IL-6) having human IL-6 cDNA linked to the H-2L^{d} promoter (Suematsu et al. Proc. Natl. Acad. Sci. U.S.A. 86:7547, 1989) was injected into the pronucleus of a fertilized egg of a C57BL/6J (B6) mouse (Nihon Clea) by microinjection according to the method described in Yamamura et al., J. Biochem. 96:357, 1984.

The fertilized egg was transplanted to the oviduct of a female ICR mouse that had been subjected to pseudogestation treatment. Thereafter for the newborn mouse, the integration of hIL-6 cDNA was screened by Southern blot analysis of the EcoRI-digested tail DNA using as the probe ³²P-labelled TaqI-BanII fragment of human IL-6 cDNA. The animals that tested positive for the integration were bred with a B6 mouse to establish a line of the mouse having the same genotype.

### Reference Example 2. Preparation of rat anti-IL-6R antibody

CHO cells producing mouse soluble IL-6R were prepared as set forth by Saito et al., J. Immunol. 147:168-173, 1991. The cells were incubated in αMEM containing 5% fetal bovine serum (FBS) at 37°C in a humidified air containing 5% CO₂. The conditioned medium was recovered and was used as a preparation of mouse sIL-6R. The concentration of mouse sIL-6R in the medium was determined by a sandwich ELISA using monoclonal anti-mouse IL-6R antibody RS15 (Saito et al., J. Immunol. 147:168-173, 1991) and rabbit polyclonal anti-mouse IL-6R antibody.

Mouse sIL-6R was purified from the mouse sIL-6R preparation using an affinity column that had been adsorbed with monoclonal anti-mouse IL-6R antibody (RS12). Fifty micrograms of purified mouse sIL-6R in complete Freund's adjuvant was subcutaneously injected to a Wistar rat and then the animal was boosted for four times with subcutaneous injection of 50 µg of mouse sIL-6R in incomplete Freund's adjuvant once per week from after two weeks. At one week after the first booster injection, the rats were intravenously administered with 50 µg of mouse sIL-6R in 100 µl of phosphate buffered saline (PBS).

Three days later, the spleen was removed from the rats and the rats' splenocytes were subjected to fusion treatment with mouse p3Ul myeloma cells at a ratio of 10 : 1. The cells were incubated at 37°C overnight in 100 µl of RPMI 1640 medium containing 10 % FBS in wells of 96-well plates (Falcon 3075), and then 100 µl of a medium containing hypoxanthine/aminopterin/thymidine (HAT) was added thereto. A half of the medium was daily replaced by the HAT medium for four days.

Seven days later, a hybridoma that produces anti-mouse sIL-6R was selected by a mouse sIL-6R binding assay (ELISA). Briefly, 100 µl of the culture supernatant of the hybridoma was incubated in a plate previously coated with 1 µg/ml of rabbit polyclonal anti-rat IgG antibody. The plate was washed and then was incubated with 100 µg/ml of mouse sIL-6R. After washing, rabbit polyclonal anti-mouse IL-6R antibody was added to 2 µg/ml, the plate was washed, and then was incubated with alkaline phosphatase-conjugated goat polyclonal anti-rabbit IgG antibody (Tago) for 60 minutes.

Finally, after washing, the plate was incubated with a substrate of alkaline phosphatase (Sigma 104;

p-nitrophenyl phosphate) and read at 405 nm using a plate reader (Toso). The hybridoma that recognizes mouse sIL-6R was cloned twice by the limiting dilution method. For preparation of ascites, a BALB/c nu/nu mouse was injected twice with 0.5 ml of pristane and three days later 3 x 10⁶ cells of the established hybridoma cells were injected intraperitoneally. Ten to 20 days later, the ascites was collected and a monoclonal antibody MR16-1 was purified therefrom using a protein G column (Oncogene Science).

The neutralizing effect on IL-6 of the antibody produced by MR16-1 was tested by incorporation of ³H-thymidine by MH60.BSF2 cells (Matsuda et al., Eur. J. Immunol. 18:951-956, 1988). MH60.BSF2 cells were aliquoted in an amount of 1 x 10⁴ cells/200 µl/well into the 96-well plate and then mouse IL-6 (10 pg/ml) and MR16-1 or RS12 antibody were added to the wells followed by incubation of the cells at 37°C in a 5% CO₂ for 44 hours. Subsequently ³H-thymidine (1 mCi/well) was added to each well and, four hours later, tested for incorporation of ³H-thymidine.

### Example 1.

Thirty one transgenic mice having human IL-6 cDNA that were reproduced from the B6 IL-6 transgenic mouse (B6 IL-6 Tgm) prepared in reference example 1, and 11 normal littermates having no human IL-6 cDNA were used (both are 4-week old; male). B6 IL-6 Tgm were divided into five groups (Group 1 to Group 5) of six animals per each group and only Group 1 consisted of seven animals. The normal littermates were divided into Group 6 of 5 mice and Group 7 of six mice.

The administration schedule was as follows:
Group 1 (B6 IL-6 Tgm): At 4-week old (the first day of the experiment), rat IgGl_antibody (KH5) (control antibody) was intravenously injected at a dose of 2 mg/0.2 ml, and at 5-week old (day 8 of the experiment) and after, 100 µg of KH5 antibody was subcutaneously injected twice every week (every three to four days).
Group 2 (B6 IL-6 Tgm): At 4-week old, MR16-1 antibody was intravenously injected at a dose of 2 mg/0.2 ml, and at 5-week old and after, 100 µg of MR16-1 was subcutaneously injected twice every week.
Group 3 (B6 IL-6 Tgm): At 4-week old, 0.2 ml of phosphate buffered saline was intravenously injected, and at 5-week old and after, 100 µg of MR16-1 was subcutaneously injected twice every week.
Group 4 (B6 IL-6 Tgm): At 4-week old, 2 mg/0.2 ml of MR16-1 was intravenously injected, and at 5-week old and after, 400 µg of MR16-1 was subcutaneously injected once every other week.
Group 5 (B6 IL-6 Tgm): At 4-week old, 2 mg/0.2 ml of MR16-1 was intravenously injected, and at 5-week old and after, 1 mg of MR16-1 was subcutaneously injected every other week.
Group 6 (B6 normal littermates): At 4-week old, 2 mg/0.2 ml of the control antibody KH5 was intravenously injected, and at 5-week old and after, 100 µg of KH5 was subcutaneously injected twice every week.
Group 7 (B6 normal littermates): At 4-week old, 2 mg/0.2 ml of MR16-1 was intravenously injected, and at 5-week old and after, 100 µg of MR16-1 was subcutaneously injected twice every week.

The test methods used herein are as follows:
Measurement of body weight and determination of urinary protein: Measurement of body weight and determination of urinary protein by urinary protein test paper (Combistics Sankyo) were carried out every week. The readings of urinary protein of three plus (100 to 300 mg/dl) or higher were taken as positive.

Collection of blood: Blood was collected from the retro-orbital sinus every other week from the start of the experiment (4-week old) and the total blood was collected from vena cava inferior at the end of the experiment (18-week old).

Blood cell counts: Using the micro cell counter (Sysmex F-800), counts of white blood cells (WBC), red blood cells (RBC), and platelets (PLT), as well as the amount of hemoglobin (HGB) were determined. At the end of the experiment, blood smears were prepared for certain groups (Group 1, 2, 6, and 7) and differential white blood cell counts were calculated as a percentage.

Determination of IgGl concentration in the blood: It was measured by a mouse IgGl-specific ELISA using as the standard a myeloma protein.

Determination of IL-6 concentration in the blood: It was measured by a hIL-6-specific ELISA.

Determination of titer of anti-rat IgG antibody (IgG class) in the blood: Since the antibody administered is a heterogeneous antibody to the mouse, the production of antibody to the antibody given was measured by an ELISA using a rat IgG as an antigen. A result was expressed as units using as the standard IL-6 Tgm serum of an adult animal that was given the rat antibody.

Determination of blood chemical parameters: On the sera of the mice in Groups 1, 2, 3, 6, and 7 at the end of the experiment, total protein (TP), albumin (Alb), glucose (Glu), triglyceride (TG), creatinine (CRE), blood urea nitrogen (BUN), calcium (Ca), alkaline phosphatase (ALP), glutamine-pyruvate transaminase (GOT), and glutamate-pyruvate transaminase (GPT) were measured using an autoanalyzer (COBAS FARA II, Roche).

FACS analysis of bone marrow and splenocytes: At the end of the experiment, bone marrow and splenocytes were obtained from one animal each of Groups 1, 2, 6, and 7, and were subjected to analysis of cell surface antigens by the FACScan (Beckton Dickensian). The antibodies used are antibodies (Pharmingen) directed, respectively, to Gr-1 (bone marrow cells), CD4, CD8, and B220 (splenocytes).

Autopsy: At the end of the experiment, autopsy was carried out and the weight of the spleen was measured and major organs were visually inspected.

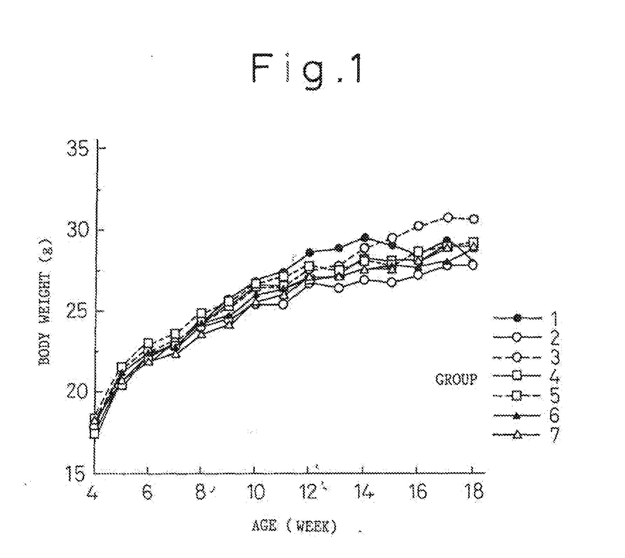

Body weights: Changes in body weights of each group were shown in Fig. 1. There was an increase in the weights in Groups 1 and 3. No difference was observed in changes in body weights among other groups.

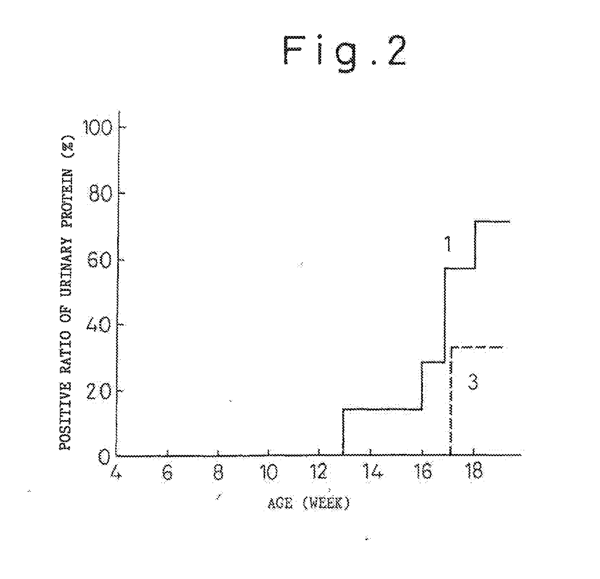

Urinary protein: In Group 1 urinary protein-positive animals began to appear from 13-week old (Fig. 2), and four (two at 16-week old, and 2 at 17-week old) out of seven animals died by the time of autopsy. However, no deaths were observed in the other groups. In Group 3 also, two out of six animals became positive for urinary protein by the end of the experiment, but no animals tested positive in the other groups.

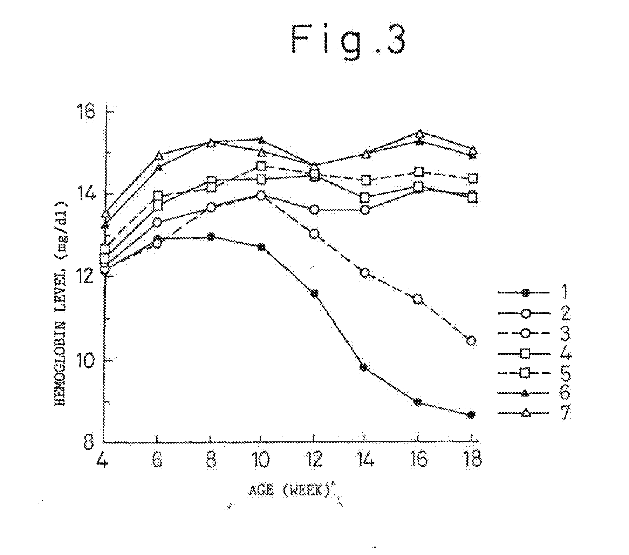

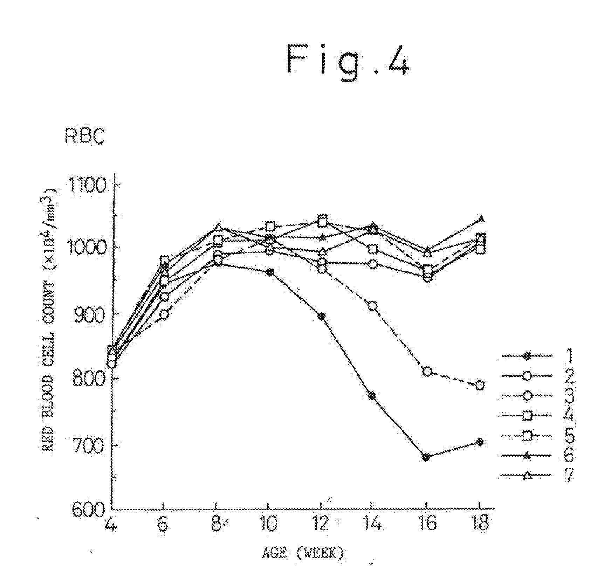

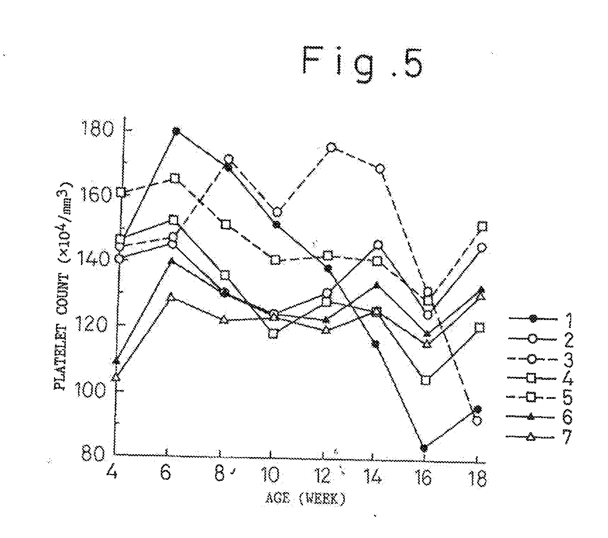

Hematological findings: In Group 1, reduction in the level of hemoglobin (Fig. 3) and RBC counts (Fig. 4) was observed, the degree becoming severe with aging. The platelet counts (Fig. 5) showed a transient increase but rapidly decreased thereafter. In Group 3, a similar tendency was observed though it was a little delayed than Group 1. On the other hand, there were neither decrease in the level of hemoglobin and in RBC nor an increase in platelet counts and subsequent decrease in any of the Groups 2, 4, and 5. In observation of differential blood cell counts of the blood smears, Group 1 has shown an elevation in neutrophils and monocytes and relevant decreases in lymphocyte fraction were observed but Group 2 has shown normal values (Table 1). Also, there was no significant difference between Groups 6 and 7.

**Table 1**

| Group | | Juvenile neutrophils | Mature neutrophils | Eosinophils | Basophils | Monocytes | Lymphocytes | Others |
|---|---|---|---|---|---|---|---|---|
| 1 | Mean SD* | 2.00 | 31.33 | 1.33 | 0.00 | 9.33 | 56.00 | 0.00 |
| | | 2.00 | 3.79 | 0.58 | 0.00 | 4.93 | 9.54 | 0.00 |
| 2 | Mean SD* t-test | 0.33 | 13.83 | 2.33 | 0.00 | 2.00 | 81.00 | 0.50 |
| | | 0.52 | 4.17 | 1.03 | 0.00 | 2.28 | 4.82 | 0.55 |
| | | 0.0676 | 0.0000 | 0.0557 | | 0.0129 | 0.006 | 0.0676 |
| 6 | Mean SD* | 0.30 | 14.10 | 2.80 | 0.00 | 1.30 | al.40 | 0.10 |
| | | 0.45 | 4.60 | 0.91 | 0.00 | 1.04 | 4.08 | 0.22 |
| 7 | Mean SD* t-test | 0.42 | 10.67 | 2.42 | 0.08 | 0.58 | 85.75 | 0.08 |
| | | 0.38 | 2.32 | 0.97 | 0.20 | 0.49 | 1.92 | 0.20 |
| | | 0.6484 | 0.1406 | 0.5101 | 0.3816 | 0.1644 | 0.0427 | 0.8992 |

| | | | | | | | | |
|---|---|---|---|---|---|---|---|---|
| *SD: Standard deviation | | | | | | | | |

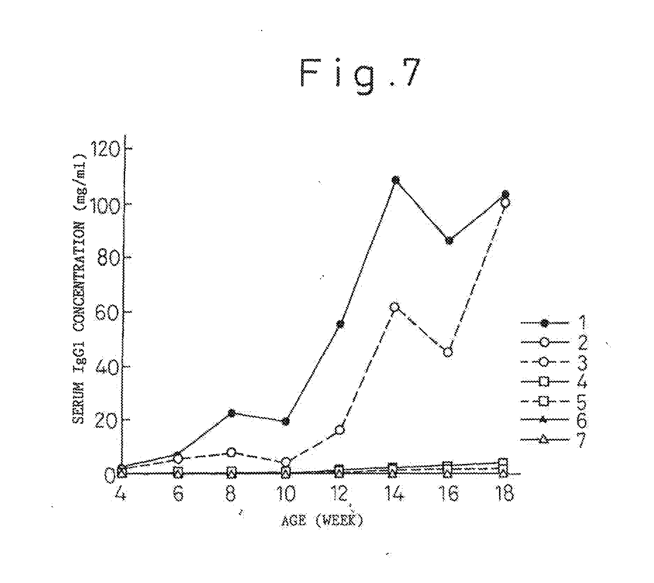

IgGl concentration in the blood: In Group 1, IgGl concentration in blood has shown a remarkable increase from immediately after the start of the experiment, finally reaching about 100 times the concentration of the normal mice (Fig. 7). In group 3, increases in IgGl concentration were noted a little later than in Group 1. In contrast, there was no increase in IgGl concentration in Groups 2, 4, and 5, staying at almost the same level during the experiment. On the other hand, no change related to antibody administration was observed in the normal mice.

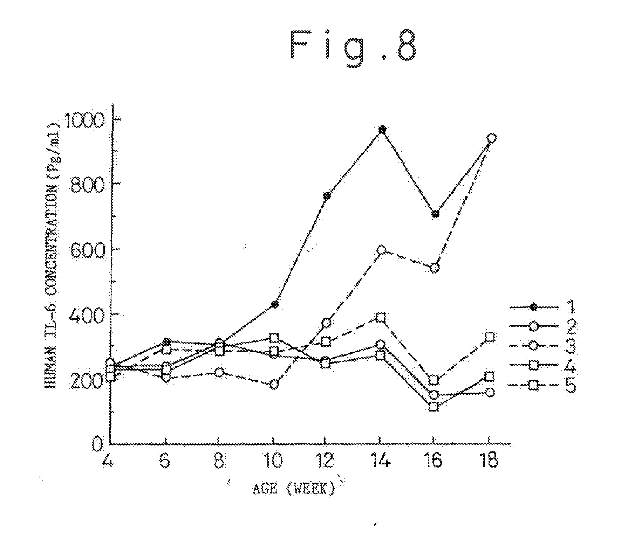

hIL-6 concentration in the blood: hIL-6 concentration in the blood (Fig. 8) varied in the same manner as the IgGl, showing increases in groups 1 and 3, whereas staying at almost the same level in the other groups during the experiment.

Titer of anti-rat IgG antibody in the blood: Antibody against anti-rat IgG was detected in Group 1, 3, and 6 (Table 2). All the animals in Group 1 and 3 have shown a high titer, whereas in Group 6 only two out of 5 animals have shown an increase in titer. On the other hand, there was no significant increase observed in the other groups.

**Mouse anti-rat antibody (units/ml) Table 2**

| Group | Age (week) 4 | 6 | 8 | 10 | 12 | 14 | 16 | 18 |
|---|---|---|---|---|---|---|---|---|
| 1 | 0.15 | 0.78 | 1.69 | 7.41 | 100< | 100< | 100< | 100< |
| 2 | 0.22 | 0.34 | 0.45 | 0.38 | 0.43 | 0.50 | 0.39 | 0.30 |
| 3 | 0.14 | 0.61 | 0.69 | 0.67 | 2.27 | 4.74 | 14.25 | 41.24 |
| 4 | N.D. | N.D. | N.D. | N.D. | N.D. | N.D. | N.D. | 0.57 |
| 5 | N.D. | N.D. | N.D. | N.D. | N.D. | N.D. | N.D. | 0.28 |
| 6 | N.D. | N.D. | N.D. | N.D. | N.D. | N.D. | N.D. | 3.55 |
| 7 | N.D. | N.D. | N.D. | N.D. | N.D. | N.D. | N.D. | 0.20 |

| | | | | | | | | |
|---|---|---|---|---|---|---|---|---|
| N.D.: Not determined | | | | | | | | |

Determination of blood chemical parameters: In Groups 1 and 3, there was an increase in TP and a decrease in Alb. TG and ALP were decreased in Groups 1 and 3, and even Glu was decreased in Group 1. No such changes were observed in Group 2.

**Table 3**

| Group | | TP(g/dl) | Alb(g/dl) | Glu(mg/dl) | TG(mg/dl) | CRE (mg/dl) | BUN(mg/dl) | Ca(mg/dl) | ALP(U/l) | GOT (IU/l) | GPT (U/l) |
|---|---|---|---|---|---|---|---|---|---|---|---|
| 1 | Mean | 14 | 2.41 | 77.4 | 20.7 | 0.4 | 34.8 | 8.6 | 27.67 | 33.33 | 6 |
| | SD | 1.33 | 0.3 | 14.5 | 8.33 | 0.2 | 23.7 | 0.35 | 5.69 | 5.86 | 1.73 |
| 2 | Mean | 5.68 | 3.31 | 199 | 62.3 | 0.51 | 23.3 | 8.67 | 156.5 | 37.5 | 5.6 |
| | SD | 0.3 | 0.2 | 29.5 | 10.9 | 0.22 | 4.08 | 0.58 | 14.31 | 8.22 | 1.14 |
| 3 | Mean | 12.6 | 2.92 | 253 | 41 | 0.61 | 26.4 | 9.82 | 54.17 | 27.33 | 6.83 |
| | SD | 2.85 | 0.64 | 60.2 | 16.3 | 0.17 | 6.25 | 0.83 | 42.62 | 5.65 | 1.72 |
| 6 | Mean | 5.9 | 3.84 | 289 | 105 | 0.87 | 27.9 | 8.9 | 181 | 33.2 | 9.6 |
| | SD | 0.65 | 0.46 | 98.9 | 28.8 | 0.22 | 6.32 | 0.74 | 21.24 | 8.9 | 5.81 |
| 7 | Mean | 5.86 | 3.63 | 300 | 94 | 0.75 | 26.8 | 8.98 | 196.83 | 34.5 | 6.5 |
| | SD | 0.53 | 0.34 | 25.5 | 20 | 0.2 | 5.26 | 0.82 | 21.68 | 4.89 | 3.08 |

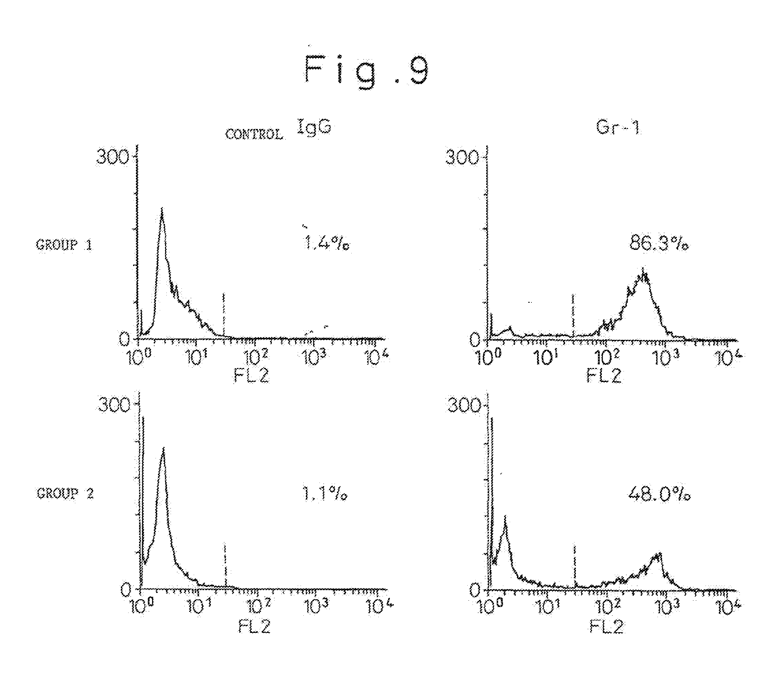

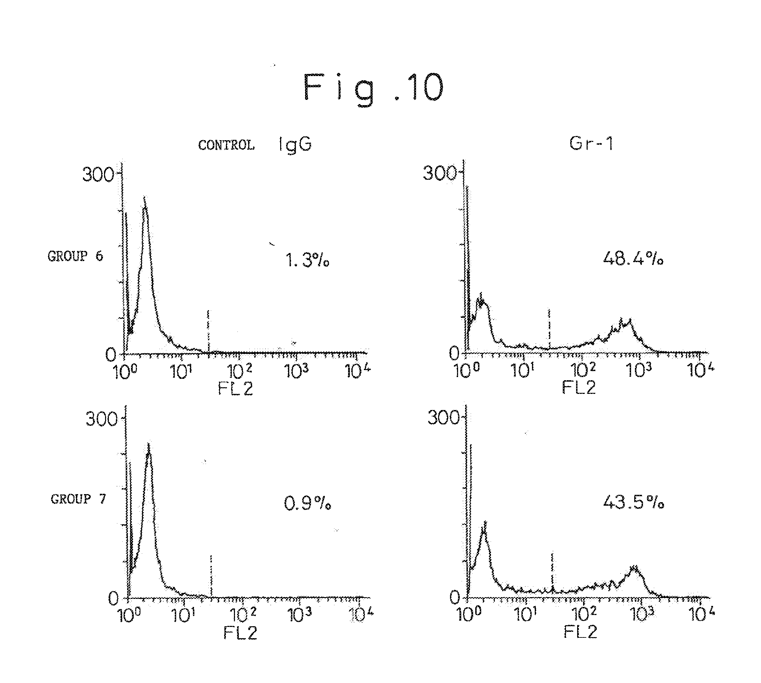

FACS analysis: Analysis on bone marrow cells (BM) and splenocytes (sp) of Groups 1, 2, 6 and 7 revealed that there was an extreme increase in the ratio of Gr-1 positive cells which are granulocytic precursor cells, in the BM cells in Group 1 (Fig. 9, and Fig. 10), but those in Group 2 have shown similar values to normal littermates. There was substantially no difference between Groups 6 and 7. With regard to the ratio of CD4-, CD8-, and B220-positive cells in sp, there were no differences between the groups except that in Group 1 CD8- and B220-positive cells were decreased due to an increase in plasma cells (Table 4)

**Table 4**

| Analysis of surface antigen of splenocytes | | | |
|---|---|---|---|
| Group | CD4⁺ | CD8⁺ | B220⁺ |
| 1 | 13.2% | 5.4% | 23.1% |
| 2 | 18.5% | 14.3% | 50.0% |
| 3 | 19.9% | 15.0% | 53.1% |
| 4 | 13.9% | 10.6% | 57.3% |

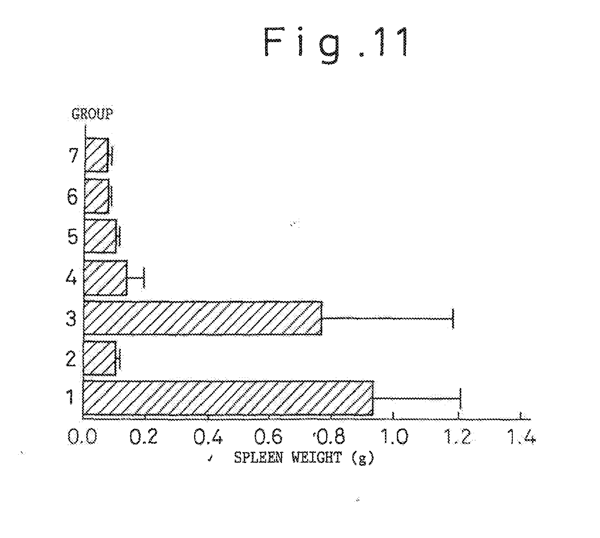

Autopsy findings: In Group 1 and 3, swelling of systemic lymph nodes and enlargement of the spleen were conspicuous (Fig. 11) and so was decoloration of the kidney. Partly, enlargement of the liver was also noted. These changes were not observed in the other groups, and there was no remarkable changes except that in Group 2, 4, and 5 slight enlargement of the spleen was noted as compared to the normal littermates.

The results of this experiment will now be explained. In the IL-6 Tgm (Group 1) that had been administered the control antibody, a variety of symptom were observed such as IgGl plasmacytosis, anemia, thrombocytosis, thrombocytopenia, renal failure, abnormal blood chemical parameters, etc. However, it became apparent that these symptoms can be completely suppressed by MR16-1.

It is known that IL-6 causes B cells to terminally differentiate into plasma cells [Muraguchi, A. et al., J. Exp. Med. 167:332-344, 1988], and in the case of IL-6 Tgm, IL-6 production caused an increase in IgGl concentration in the blood and an increase in TP concentration and decrease in Alb concentration in the serum. These facts indicate the onset of IgGl plasmacytosis has taken place.

Remarkable enlargement of systemic lymphatic tissues such as the lymph nodes and the spleen caused by this would be responsible for an increase in body weight in spite of the aggravation of the general conditions caused by progression of the disease in Groups 1 and 3. MR16-1 not only suppressed these conditions completely but also suppressed the increase of IL-6 concentration in the blood. Thus, it was confirmed that the increase in IL-6 concentration in the blood associated with aging as observed with I1-6 Tgm is directly related to the progress of plasmacytosis. It was believed, therefore, that the proliferated plasma cells themselves actively produce IL-6 which further increase the growth of the plasma cells, with a result that IL-6 is produced in large amounts.

As the effects of IL-6 on the hemocytes, the effect of increasing platelets [Ishibashi, T. et al., Proc. Natl. Acad. Sci. U.S.A. 86:5953-5957, 1989; Ishibashi, T. et al., Blood 74:1241-1244, 1989] and the effect of inducing macrocytic anemia [Hawley, R.G. et al., J. Exp. Med. 176:1149-1163, 1992] are known. In addition to the above, in IL-6 Tgm, there is observed thrombocytopenia associated with aging which is believed to be autoimmunity related to polyclonal B cell activation [Miyai, Tatsuya et al., ibid].

MR16-1 completely inhibited the direct and indirect effects of IL-6 on the hemocyte, but did not affect the blood cell counts of the normal littermate. Thus, it was confirmed that anti IL-6 receptor antibody does not affect the hematocytes at all. In IL-6 Tgm, there were observed the increases in the ratio of Gr-1-positive cells, which are considered as granulocytic precursor cells and in the ratio of peripheral neutrophils. Though IL-6 is known to increase neutrophils, its detailed mechanism has not been clarified yet. It was found out in this study that this effect is a phenomenon taking place at the level of the precursor cells in the bone marrow. In this study also, it was found out that MR16-1 completely suppressed the effects of IL-6 but did not affect the level of the neutrophils in the bone marrow and the peripheral blood.

MR16-1 also suppressed the onset of nephritis observed in IL-6 Tgm. It has been reported that IL-6 is closely related to the onset of mesangium proliferative nephritis as an autocrine growth factor of the mesangium cells. Although nephritis in IL-6 Tgm has also been confirmed to be a mesangium proliferative nephritis, the involvement of the immune system enhanced by IL-6 cannot be denied [Katsume, Asao et al., a presentation at the 21st Meeting of Japan Immunology Society, "Characterization of SCID x (SCID x H-2Ld hIL-6 transgenic mice)," 1991]. In any way, since there was suppression on the appearance of urinary protein and on deaths, it was made clear that anti-IL-6 receptor antibody is effective for suppressing the onset of nephritis caused by IL-6 production.

In IL-6 Tgm, there was observed a significant reduction in serum Glu and Tg concentrations which are indicators for cachexia. In the present experiment, the administration of MR16-1 antibody was found to be effective for ameliorating cachexia because Glu and Tg values were decreased in Group 1 while in group 2 these values returned to almost the same level as the normal. Since MR16-1 is a rat IgGl, a heteroprotein to mice, it is easily anticipated that antibodies against the administered antibody may be produced which would make the antibodies given ineffective.

In an attempt to induce immunological tolerance by exposing to a large quantity of antigen at the first sensitization in the present experiment, groups were set up that were intravenously given 2 mg/mouse of antibody at the first administration. Among the MR16-1 administration groups, the groups that were subjected to this treatment (Group 1, 4, and 5) produced no detectable anti-rat IgG antibody regardless of the interval and the dose of administration, leading to complete suppression of the onset of the disease. But Group 3 have eventually shown same symptoms as Group 1 which is the control antibody administration group though the group has shown an increase in anti-rat IgG antibody and the onset of the disease was slightly delayed than Group 1.

Therefore, it is believed that the treatment was effective for inducing immunological tolerance, but the anti-rat IgG antibody was also detected in all animals of Group 1 and 2/5 animals of Group 6 that were given the control antibody in the same schedule. Since the progress of plasmacytosis induces polyclonal B cell activation in IL-6 Tgm, it cannot be concluded that the anti-rat IgG antibody detected in Group 1 and 3 is an antibody specific for the given antibody. However, it was inferred that the inducing effect of immunological tolerance by being exposed to a large quantity of antigen at the first sensitization in Groups 2, 4, and 5 combined with the inhibiting effect of production of specific antibodies due to administration of a large quantity of MR16-1 served to induce complete tolerance.

It was clarified in the present experiment that anti- IL-6 receptor antibody is extremely effective against a variety of diseases caused by IL-6 production without affecting the normal level.

### Example 2.

The effect of mouse IL-6 receptor antibody on the colon 26-induced cachexia model was investigated. The mice used were 6-week old male BALB/c mice, to which a 2 mm block of colon 26 was subcutaneously implanted into the latus of the mouse on the first day of the experiment. The mouse IL-6 receptor antibody MR16-1 (see reference example 2) was intravenously given at a dose of 2 mg/mouse immediately before the implantation of colon 26 on the first day of the experiment and then subcutaneously given at a dose of 0.5 mg/mouse on day 7, 11, 14, and 18 (n=7). It has already been confirmed in the previous experiment that neutralizing antibodies against the heteroprotein do not easily appear in this method. To the tumor-bearing control group, the rat IgGl control antibody (KH5) was administered in a same schedule (n=7). Furthermore, a PBS administration group was set up as a non-tumor-bearing control group (n=7). After the start of the experiment, body weight was measured every day and blood chemical parameters and the concentrations of ionized calcium in the blood were measured on day 11 and 15 after the start of the experiment.

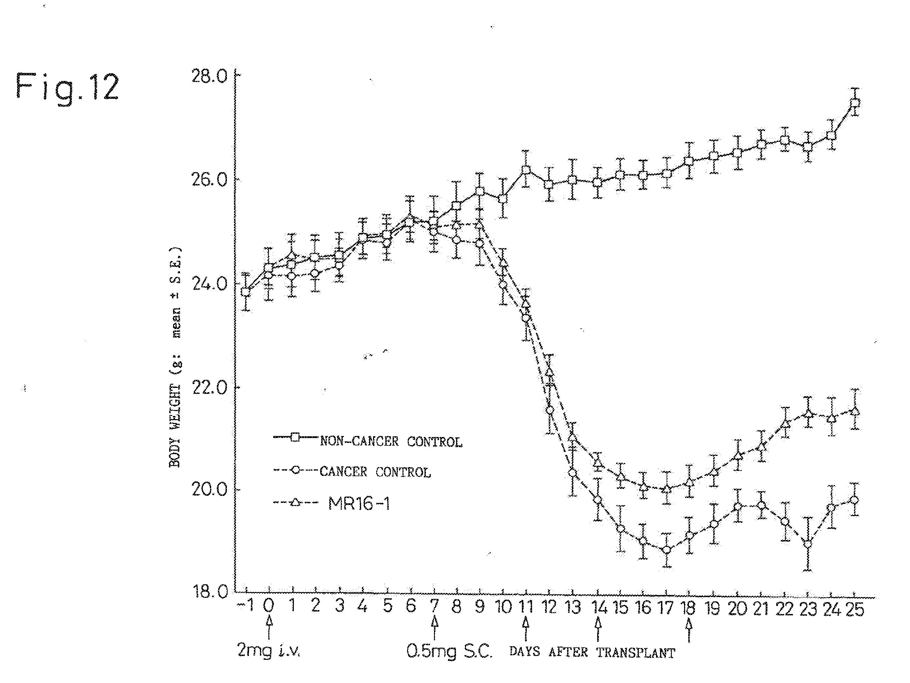

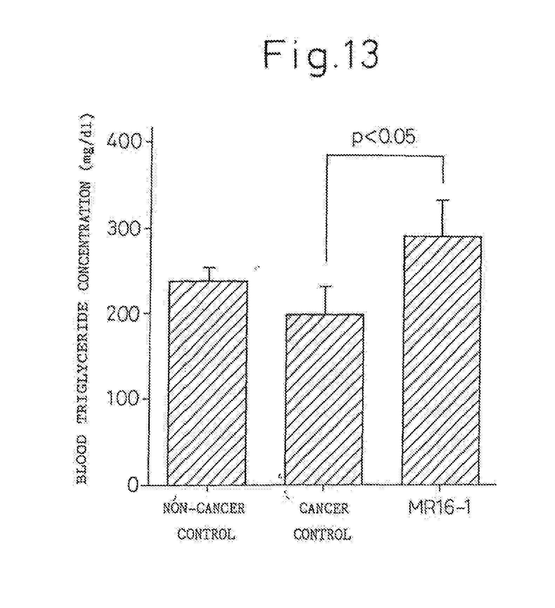

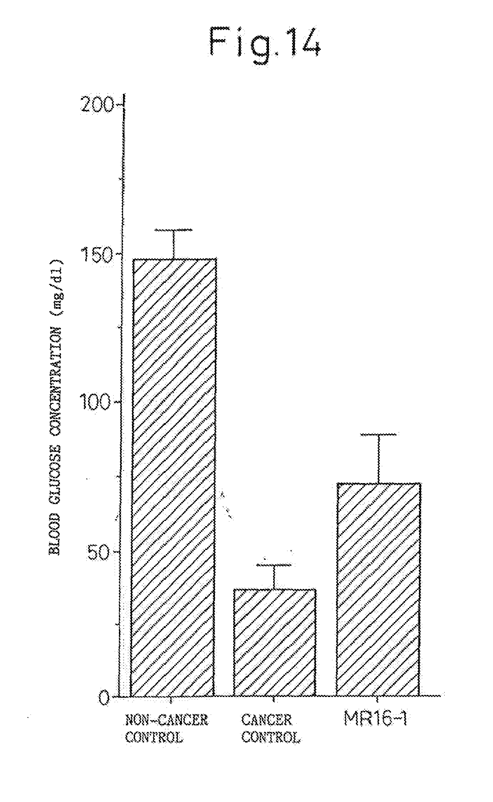

There was a remarkable reduction in body weight in the tumor-bearing group on day 10 and after as compared to the non-tumor-bearing group, whereas a partial effect of suppressing the reduction in body weight was exhibited in the MR16-1 administration group (Fig. 12). The concentration of triglyceride in the blood on day 11 and that of glucose in the blood on day 15 are shown, respectively, in Fig. 13 and Fig. 14. These values were remarkably reduced in the tumor-bearing control group as compared to the non-tumor-bearing control group, while in the MR16-1 administration group, a suppressing tendency for glucose and a significant suppressing effect for triglyceride were observed.

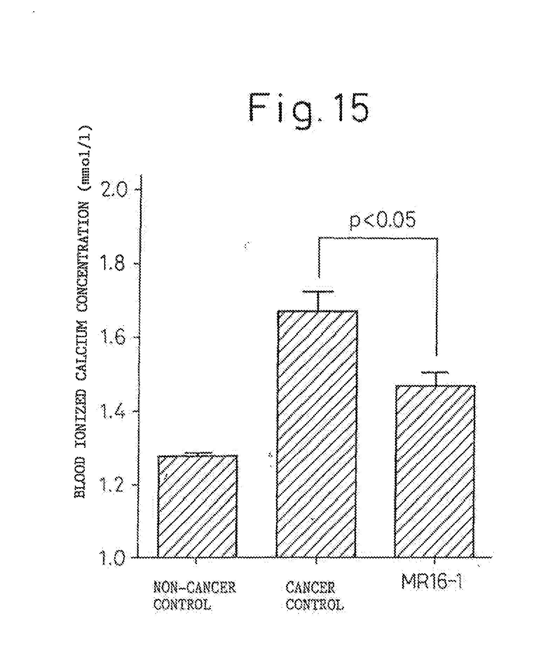

The concentration of ionized calcium in the blood on day 11 was remarkably elevated in the tumor-bearing control group as compared to the non-tumor-bearing control group, whereas in the MR16-1 administration group a significant suppressing effect was observed (Fig. 15).

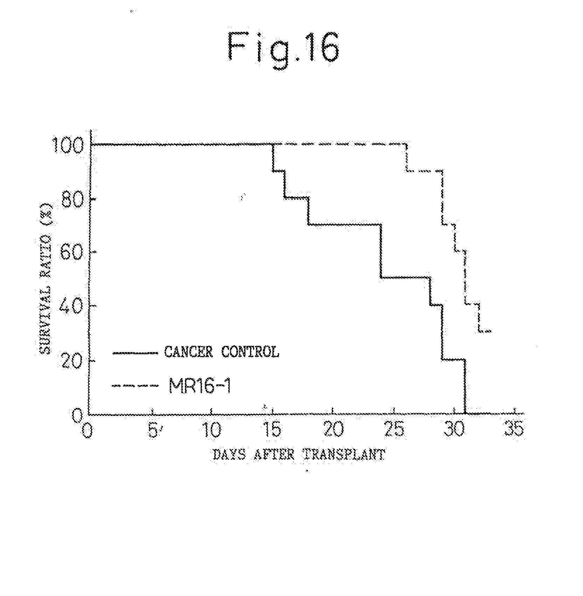

An experiment to confirm an effect on survival time was carried out in a similar schedule as above (n=10). As a result, an effect on survival time was observed in the MR16-1 administration group (Fig. 16).

### Example 3.

The effect of IL-6 receptor antibody on the occ-1-induced cachexia model accompanied by hypercalcemia was investigated. The mice used were 6-week old male nude mice. On the first day of the experiment, squamous carcinoma cell line, occ-1, was subcutaneously implanted into the latus of the mouse. The mouse IL-6 receptor antibody MR16-1 (see reference example 2) was given intravenously at a dose of 2 mg/mouse immediately before the implantation of occ-1 on the first day of the experiment and then 100 µg/mouse was subcutaneously given on day 7 and 10 (n=6). It has already been confirmed in the previous experiment that neutralizing antibodies against the heteroprotein, rat antibody, do not easily appear in this method. To the tumor-bearing control group the rat IgGl control antibody (KH5) was administered in a same schedule (n=6). Furthermore, a PBS administration group was set up as a non-tumor-bearing control group (n=7). After the start of the experiment, body weight and the concentrations of ionized calcium in the blood were measured on day 10 and 12 after the start of the experiment.

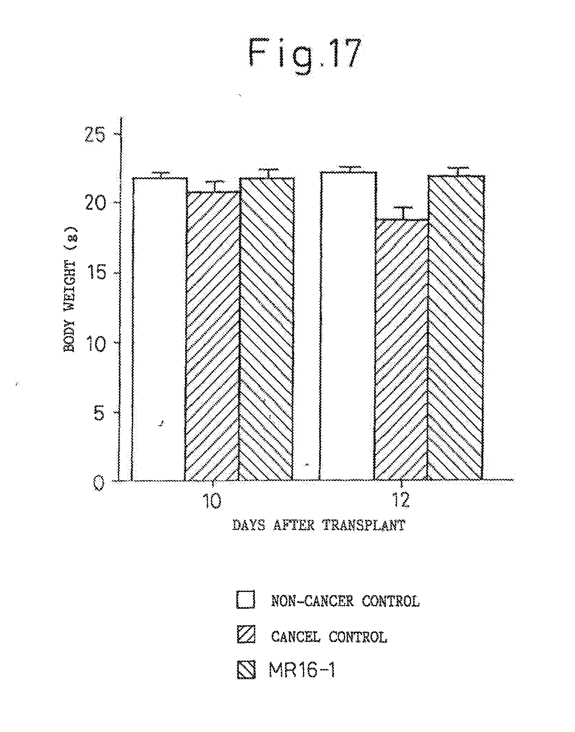

There was a reduction in body weight in the tumor-bearing group but the MR16-1 administration group has shown a similar change in body weight as the non-tumor-bearing control group, indicating suppression of reduction in body weight (Fig. 17).

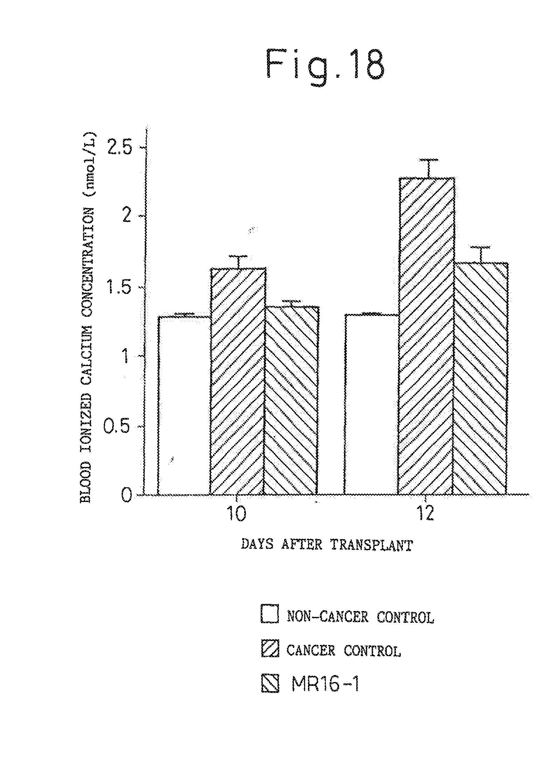

The concentration of ionized calcium in the blood was remarkably elevated in the tumor-bearing control group as compared to the non-tumor-bearing control group, whereas in the MR16-1 administration group the elevation was strongly suppressed (Fig. 18).

The following numbered paragraphs assist further in describing the invention and its embodiments.
1. A pharmaceutical composition for prevention or treatment of diseases caused by interleukin-6 production comprises an antibody to interleukin-6 receptor.
2. The disease caused by said interleukin-6 production may be plasmacytosis.
3. The disease caused by said interleukin-6 production may be hyperimmunoglobulinemia.
4. The disease caused by said interleukin-6 production may be anemia.
5. The disease caused by said interleukin-6 production may be nephritis.
6. The disease caused by said interleukin-6 production may be cachexia.
7. The plasmacytosis mentioned in paragraph 2 may be induced by rheumatism.
8. The plasmacytosis mentioned in paragraph 2 may be induced by Castleman's disease.
9. The nephritis mentioned in paragraph 5 may be mesangium proliferative nephritis.
10. The said antibody may be a monoclonal antibody.
11. The antibody of paragragh 10 may be PM-1 antibody.
12. The antibody of paragragh 10 may be a chimeric antibody.
13. The antibody of paragragh 10 may be a reshaped human antibody.
14. The antibody of paragragh 13 may be a reshaped human PM-1 antibody.

## Claims

1. A pharmaceutical composition for prevention or treatment of nephritis or cachexia caused by interleukin-6 production, comprising an antibody to interleukin-6 receptor.

2. A pharmaceutical composition for prevention or treatment according to claim 1, wherein said nephritis is mesangium proliferative nephritis.

3. A pharmaceutical composition for prevention or treatment according to claim 1 or 2, wherein said antibody is a monoclonal antibody.

4. A pharmaceutical composition for prevention or treatment according to claim 3, wherein said antibody is PM-1 antibody.

5. A pharmaceutical composition for prevention or treatment according to claim 3, wherein said antibody is a chimeric antibody.

6. A pharmaceutical composition for prevention or treatment according to claim 3, wherein said antibody is a reshaped human antibody.

7. A pharmaceutical composition for prevention or treatment according to claim 6, wherein said antibody is a reshaped human PM-1 antibody.
